# DEMANDE DE BREVET EUROPEEN

(11) **EP 1 479 367 A1**
(43) Date de publication de la demande: **24.11.2004**
(21) Numéro de dépôt: 04291213.9
(22) Date de dépôt: 12.05.2004
(51) Int. Cl.: A61K 7/043, A61K 7/032, A61K 7/09, A61K 7/11, A61K 7/075

(54) **Utilisation d'un extrait de bactérie filamenteuse non photosynthéthique non fructifiante pour le renforcement des matières kératiniques**

(30) Priorité: 19.05.2003 FR 0305974
(71) Demandeur: L'OREAL, 75008 Paris (FR)
(72) Inventeur: Lalaudière, Christophe, 37100 Tours (FR); Martin, Richard, 37210 Rochecorbon (FR)
(74) Mandataire: Allab, Myriam

(57) **Abrégé**

L'invention concerne l'utilisation, dans une composition cosmétique, d'une quantité efficace d'au moins un extrait de bactérie filamenteuse non photosynthétique non fructifiante, en tant qu'agent améliorant la structure des matières kératiniques.

En particulier, l'utilisation vise notamment à (i) durcir les ongles, augmenter leur épaisseur, stimuler leur vitesse de pousse et/ou de repousse, augmenter leur résistance à la cassure et/ou au dédoublement ; ou à (ii) augmenter l'élasticité et/ou maintenir la mise en forme des cheveux et/ou des cils.
La bactérie filamenteuse non photosynthétique non fructifiante est préférentiellement *Vitreoscilla filiformis.*

L'invention porte également sur des compositions cosmétiques adaptées respectivement à la mise en forme et/ou au coiffage des cheveux, ou au soin et/ou au maquillage des cils ou des ongles comprenant ledit extrait bactérien, ainsi que sur un procédé cosmétique de mise en forme et/ou de coiffage des cheveux, de soin et/ou de maquillage des cils ou ongles.

## Description

L'invention concerne l'utilisation, dans une composition cosmétique, d'une quantité efficace d'au moins un extrait de bactérie filamenteuse non photosynthétique non fructifiante, en tant qu'agent améliorant la structure des matières kératiniques.
En particulier, l'utilisation vise notamment à (i) durcir les ongles, augmenter leur épaisseur, stimuler leur vitesse de pousse et/ou de repousse, augmenter leur résistance à la cassure et/ou au dédoublement et améliorer leur état général ; ou à (ii) augmenter l'élasticité et/ou maintenir la mise en forme des cheveux et/ou des cils.
La bactérie filamenteuse non photosynthétique non fructifiante est préférentiellement *Vitreoscilla filiformis.*

L'invention porte également sur des compositions cosmétiques particulières adaptées respectivement à la mise en forme et/ou au coiffage des cheveux, ou au soin et/ou au maquillage des cils ou des ongles comprenant ledit extrait bactérien, ainsi que sur un procédé cosmétique de mise en forme et/ou de coiffage des cheveux, ou de soin et/ou de maquillage des cils ou des ongles.
Les kératines sont des composés fondamentaux de la peau, du cheveu et de l'ongle. Ces molécules fibreuses, insolubles dans l'eau, participent à la forme du cheveu et de l'ongle ainsi qu'à leur élasticité et à leur résistance.

Il est bien connu que les ongles présentent, de façon fréquente, des défauts de structure et de consistance, ceux-ci pouvant être d'origine diverses et notamment liés au métabolisme propre de l'individu, à ses conditions de vie, à son mode d'alimentation, à son âge, à ses états de fatigue ou de surmenage.
Ces défauts peuvent également apparaître sous l'effet d'actions qui érodent, à la suite par exemple d'expositions prolongées ou répétées à des agents détergents, à des solvants, à des produits chimiques en particulier ménager, à des atmosphères chaudes ou froides, humides ou sèches, ou à des expositions aux rayonnements UV.
Ces défauts de structure et de consistance ont pour effet de rendre la surface des ongles inesthétique, ce qui peut être source de gêne et de désagréments multiples.

La kératine joue également un rôle dans l'élasticité et la forme de la fibre capillaire. Dans certains cas, le cheveu peut paraître mou et la coiffure sans tenue ; ces défauts de structure peuvent être liés, comme pour les ongles, au métabolisme propre de l'individu, à ses conditions de vie, à son mode d'alimentation, à son âge, à ses états de fatigue ou de surmenage.

Il existe donc un besoin de trouver des produits capables de modifier les propriétés physicochimiques des kératines, et influer ainsi sur l'élasticité et/ou la résistance des matières kératiniques.

Par 'matières kératiniques' selon l'invention, on entend notamment les cheveux, les cils et les ongles.

Or la Demanderesse vient de montrer qu'un extrait de bactérie filamenteuse non photosynthétique non fructifiante avait une affinité pour la kératine et était capable, après application sur des mèches de cheveux ou sur les ongles :
- de prolonger la tenue ou mise en forme de la coiffure, en maintenant plus longtemps l'effet bouclé d'une mèche de cheveu et en augmentant son élasticité ;
- de durcir les ongles,, augmenter leur résistance à la cassure et/ou au dédoublement, augmenter l'épaisseur des ongles, stimuler la pousse et/ou la repousse des ongles, et améliorer ainsi leur aspect global et la tenue des vernis à ongles.

La Demanderesse a précédemment montré l'effet immunomodulateur et apaisant dudit extrait bactérien et a décrit des compositions cosmétiques pour cils ou cheveux comprenant un extrait de bactérie filamenteuse non photosynthétique non fructifiante associé à un filtre UV organique (WO02/056858 ou des compositions pour cheveux comprenant ledit extrait associé à un produit à effet irritant (EP0761204). Mais il n'est nullement fait référence à un effet sur la structure des matières kératiniques, ni décrit les compositions particulières pour cheveux, cils et ongles objets de l'invention, mettant en oeuvre cet effet. Par ailleurs, les compositions de l'art antérieur utilisent préférentiellement le surnageant dudit extrait et non l'extrait cellulaire, tel que préféré dans le contexte de l'invention.
La demande EP0876813 (L'OREAL) concerne une composition comprenant une quantité efficace de milieu de culture d'au moins une bactérie filamenteuse non photosynthétique, qui n'est pas l'objet de la présente invention portant sur des compositions comprenant un extrait de la biomasse de la bactérie (extrait cellulaire ou surnageant dudit extrait).

L'invention concerne donc l'utilisation, dans une composition cosmétique, d'une quantité efficace d'au moins un extrait de bactérie filamenteuse non photosynthétique non fructifiante, en tant qu'agent améliorant la structure des matières kératiniques.

Par 'agent améliorant la structure' des matières kératiniques, on entend un agent améliorant les propriétés physiques et/ou mécaniques des matières kératiniques, en particulier un agent capable de favoriser la pousse, et/ou la résistance à la tension et/ou l'élasticité desdites matières kératiniques.

Selon un premier mode de réalisation, appliqué aux ongles, ledit extrait de bactérie est destiné à durcir les ongles et/ou augmenter leur résistance à la cassure et/ou au dédoublement.
En particulier, l'utilisation de cet extrait de bactérie selon l'invention permet de diminuer la fragilisation des ongles affaiblis, et notamment striés, fendus, mous ou souples, et ayant tendance à se dédoubler. Les ongles traités avec l'extrait bactérien selon l'invention sont donc plus durs et plus résistants, donc moins cassants, et ils ne se dédoublent plus et/ou ne se fendillent plus.
L'aspect global de l'ongle est ainsi amélioré. En particulier, l'ongle traité avec une composition contenant ledit extrait de bactérie est plus lisse, plus brillant, moins abîmé, moins dédoublé et moins cassant.

L'invention concerne également l'utilisation dans une composition cosmétique, d'une quantité efficace d'au moins un extrait de bactérie filamenteuse non photosynthétique non fructifiante, ledit extrait de bactérie étant destiné à augmenter l'épaisseur des ongles.

Elle porte encore sur l'utilisation dans une composition cosmétique, d'une quantité efficace d'au moins un extrait de bactérie filamenteuse non photosynthétique non fructifiante, ledit extrait de bactérie étant destiné à stimuler la pousse et/ou la repousse des ongles.
En particulier, les compositions pour ongles à base de cet extrait seront avantageuses pour favoriser la repousse des ongles lorsque ceux-ci ont été arrachés lors d'un accident ou à la suite d'une intervention chirurgicale (ongle incarné par exemple).

L'invention concerne également l'utilisation dans une composition cosmétique, d'une quantité efficace d'au moins un extrait de bactérie filamenteuse non photosynthétique non fructifiante, ledit extrait de bactérie étant destiné à prolonger la tenue des vernis à ongles. Le vernis est ainsi moins friable et plus durable.

Selon un autre mode de réalisation, appliqué aux cheveux et/ou aux cils, ledit extrait de bactérie ou ladite composition sont destinés à augmenter l'élasticité et/ou maintenir la mise en forme des cheveux et/ou des cils.
Cette mise en forme peut être due :
- pour les cheveux, à un coiffage ou à une déformation semi-permanente ou permanente ;
- pour les cils, à un recourbement en présence d'une association de composés cosmétiques conférant de bonnes propriétés de recourbement aux cils.
La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis après avoir de préférence, rincé la chevelure, à reconstituer dans un second temps lesdites liaisons disulfures en appliquant, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage, les cheveux pouvant être selon les cas, mis sous tension pendant ou en dehors du procédé de déformation.
La présence de l'extrait bactérien selon l'invention dans des compositions capillaires de déformation permanente permettra avantageusement de diminuer la concentration des actifs destinés à l'effet de déformation permanente, lesdits actifs pouvant présenter des effets néfastes (réduction de la résistance à la cassure, responsable de l'aspect rugueux du cheveu).

Le renforcement de la kératine du cheveu peut également contribuer à :
- la protection et la réparation de la fibre capillaire endommagée par les rayonnements UV ou les traitements chimiques (coloration, permanente...) par exemple ;
- l'amélioration de l'état de surface du cheveu (structure et éclat du cheveu) ;
- une meilleure adsorption d'eau et un gonflement facilité, donnant à la chevelure plus de volume.

L'extrait de bactérie utilisable dans le cadre de l'invention est préparé à partir de bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres *Beggiatoa, Vitreoscilla, Flexithrix ou Leucothrix.*
Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)
De préférence, on utilisera un extrait de *Vitreoscilla filiformis* (ATCC 15551).

Par 'extrait de bactérie' selon l'invention, on entend un extrait de la biomasse bactérienne ou toute fraction active dudit extrait, en particulier :
(i) des cellules de bactérie isolées du milieu de culture, qui ont été concentrées, par exemple par centrifugation (extrait cellulaire non stabilisé) ; ou
(ii) des cellules de bactérie concentrées (i), puis soumises à une opération de rupture des enveloppes des cellules de bactérie, par tout moyen connu de l'homme du métier, comme l'action d'ultrasons ou préférentiellement l'autoclavage (extrait cellulaire stabilisé). Par 'enveloppes', on entend paroi bactérienne et éventuellement les membranes sous-jacentes ;
(iii) le surnageant obtenu par filtration de l'extrait cellulaire stabilisé (ii),
ou toute fraction active dudit extrait. Par fraction 'active' dudit extrait, on entend toute fraction capable de renforcer et/ou améliorer les matières kératiniques selon l'invention.

Cette fraction active peut être obtenue par les méthodes classiques de fractionnement, telles que l'extraction en présence d'un solvant, la précipitation sélective ou l'ultrafiltration tangentielle (UFT) par exemple.

Ces extraits ou fractions peuvent être conservés par exemple par congélation desdits extraits ou desdites fractions et utilisés après décongélation.
On parlera plus simplement dans le reste de la description 'd'extrait cellulaire' de bactéries ((i) et (ii)) ou de 'surnageant' dudit extrait (iii).

Et de préférence, on utilisera dans le cadre de l'invention un 'extrait cellulaire' de bactéries (sous la forme (i) ou (ii)) ou une fraction active dudit extrait.

Pour préparer l'extrait de bactérie selon l'invention, on peut cultiver lesdites bactéries selon les méthodes connues de l'homme du métier, ou se reporter en particulier à la description de la demande de brevet WO-A-93-00741. On obtient un extrait cellulaire dont on peut séparer le surnageant par exemple par filtration et centrifugation. L'extrait peut être utilisé sous forme aqueuse ou sous forme lyophilisée. Le protocole est décrit plus en détail à l'exemple 1 ci-après.
Cet extrait de bactérie peut être refractionné et utilisé pur ou dilué à différentes concentrations.

Selon un mode préféré, l'extrait de bactérie ou une fraction active dudit extrait utilisé selon l'invention est en une quantité comprise entre 0.01% et 100% du poids total de la composition, préférentiellement entre 0.05 et 10% du poids total de la composition et encore plus préférentiellement entre 0.1 et 5% du poids total de la composition.
Bien e ntendu, l'homme du m étier s aura a dapter la c oncentration d udit extrait selon le mode d'application (voie topique) et l'utilisation sur cheveux et/ou cils ou ongles, sur la base des connaissances générales dans les domaines cosmétiques correspondants.

L'invention concerne également des compositions cosmétiques de formulations adaptées respectivement à la mise en forme et/ou au coiffage et/ou au maintien des cheveux, ou au soin et/ou au maquillage des cils ou des ongles, contenant au moins une quantité efficace d'un extrait de bactérie tel que défini précédemment, dans un milieu cosmétiquement acceptable et compatible avec les matières kératiniques.

D'une manière générale, un milieu compatible avec les matières kératiniques peut être anhydre ou aqueux ; il peut ainsi comprendre une phase aqueuse et/ou une phase grasse, en particulier dans les compositions pour les cils, comprenant au moins un corps gras, choisi parmi les huiles, les cires, les corps gras pâteux et leurs mélanges.

Par phase aqueuse, on entend de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en C₂ et des aldéhydes en C₂-C₄ hydrophiles.
L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

Les compositions peuvent comprendre en outre des additifs cosmétiques conventionnels tels que par exemple : un polymère filmogène, des solvants organiques, cires, huiles, charges, céramides, agents plastifiants, agents tensioactifs, agents épaississants, agents humectants, agents hydratants, agents adoucissants, agents colorants, pigments, conservateurs, parfums et leurs mélanges.

On peut également utiliser spécifiquement dans les compositions capillaires, des agents réducteurs pour la déformation permanente du cheveu, ou des polymères fixants pour le coiffage et/ou le maintien des cheveux.
Les compositions capillaires pourront comprendre en outre des agents tensioactifs, des agents conditionneurs ou traitants, des céramides, des polymères filmogènes, des agents épaississants et un agent propulseur pour le conditionnement des laques.

Les compositions de revêtement des cils qui comprennent généralement des cires, des huiles, des charges et des agents tensioactifs, comprendront en outre selon l'invention au moins un polymère filmogène pouvant conférer de bonnes propriétés de recourbement des cils.
Elles pourront également comprendre des agents plastifiants ou au moins un agent favorisant le recourbement des cils, comme une dispersion de particules multiphasées (à phase souple et phase rigide) telle que décrite par la Demanderesse dans la demande WO 03/007898. Les particules à phases rigide et souple peuvent être présentes dans la composition en une teneur allant de 0,1 % à 70 % en poids de matières sèches de particules, par rapport au poids total de la composition, de préférence allant de 0,5 % à 55 % en poids, et préférentiellement allant de 1 % à 40 % en poids.

Les compositions pour les ongles selon l'invention comprennent notamment de l'eau ou un mélange d'eau et de solvants organiques hydrophiles et au moins un polymère filmogène.
Les pigments nacrés seront préférentiellement utilisés dans les compositions destinées à être appliquées sur les ongles.

Par 'polymère filmogène', on entend un polymère apte à former à lui seul ou en présence d'un agent auxiliaire de filmification, un film continu et adhérent sur un support, notamment sur les matières kératiniques. Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.
Le polymère filmogène peut être solubilisé ou dispersé sous forme de particules dans le milieu cosmétiquement acceptable de la composition.

Lorsque la composition, en particulier sous la forme d'un vernis à ongles, comprend un milieu solvant organique, le polymère filmogène peut être choisi notamment parmi les résines alkydes, acryliques et/ou vinyliques, les polyuréthanes et les polyesters, les celluloses et dérivés cellulosiques, telles que la nitrocellulose, les esters de cellulose, tels que l'acétate de cellulose, l'acétopropionate de cellulose, l'acétobutyrate de cellulose, les résines résultant de la condensation de formaldéhyde avec une arylsulfonamide et leurs mélanges.

Lorsque la composition, telle qu'un vernis à ongle, comprend un milieu aqueux, alors le polymère filmogène est généralement présent sous la forme de particules en dispersion dans le milieu aqueux formant ainsi un latex ou pseudo latex.

Parmi les polymères filmogènes susceptibles d'être utilisés en milieu aqueux, on peut citer les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.

La dispersion peut également comprendre un polymère associatif de type polyuréthane ou une gomme naturelle, telle que la gomme xanthane.
Comme polymère en dispersion aqueuse, on peut citer les dispersions de polymères acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la Société ZENECA, DOW LATEX 432® , par la Société DOW CHEMICAL. On peut également employer des dispersions aqueuses de polyuréthane, et notamment les polyester-polyuréthanes vendus sous les dénominations «AVALURE UR-405® », « AVALURE UR-410® », « AVALURE UR-425® », « SANCURE 2060® » par la Société GOODRICH et les polyéther-polyuréthanes vendus sous les dénominations « SANCURE 878® » par la Société GOODRICH, « NEOREZ R-970® » par la Société AVECIA.

Le polymère filmogène peut être présent dans la composition selon l'invention en une teneur en matières sèches, efficace pour l'obtention d'un film allant généralement de 5 % à 60 % en poids, par rapport au poids total de la composition, et mieux de 10 % à 40 % en poids.

Comme 'solvants organiques' utilisables dans la composition de l'invention, on peut citer, outre les solvants organiques hydrophiles cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

En particulier, dans le cas d'une composition pour les ongles, telle qu'un vernis à ongles, le solvant organique peut être choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle ,
- les acétals tels que méthylal ;
- et leurs mélanges.

Ces solvants peuvent être généralement présents en une teneur allant de 0 à 90 %, de préférence de 0,1 à 90%, de préférence encore de 10 à 90% en poids, par rapport au poids total de la composition, et mieux de 30 à 90 %.

Comme 'huiles' utilisables dans les compositions de l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyltrisiloxanes, les diphényl diméthicones, les phényl diméthicones, les polyméthylphényl siloxanes ; leurs mélanges.
Ces huiles peuvent être présentes en une teneur allant de 0,01 à 90 %, et mieux de 0,1 à 85 % en poids, par rapport au poids total de la composition.

Par 'cire' au sens de la présente invention, on entend un composé lipophile solide à température ambiante (25 °C), à changement d'état solide/liquide réversible, ayant un point de fusion supérieur ou égal à 30 °C pouvant aller jusqu'à 120 °C.
Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.
Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Comme 'agent plastifiant' utilisable dans les compositions pour ongles selon l'invention, on peut citer les glycols et leurs dérivés, les esters de glycol, les dérivés de propylène glycol, les esters d'acides carboxyliques, les dérivés oxyéthylénés et leurs mélanges.
La teneur en plastifiant peut, par exemple, aller de 0.1% à 15% en poids par rapport au poids total de la composition, et de préférence de 0.5 à 10% en poids.

Comme 'agent réducteur' utilisable dans les compositions de mise en forme ou déformation permanente des cheveux, on peut citer par exemple l'acide thioglycolique, le monothioglycolate de glycérol ou de glycol, la cystéamine et ses dérivés acylés en C1-C4 tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide bêta-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)o-hydroxyalkylamides décrits dans la demande de brevet EP 354 835 et les N-mono- ou N,N-dialkylmercapto 4-butyramides décrits dans la demande de brevet EP 368763, les aminomercaptoalkylamides décrits dans la demande de brevet EP 403 267 et les alkylaminomercaptoalkylamides décrits dans la demande de brevet EP 432000.
Lorsque les compositions selon l'invention contiennent au moins un agent réducteur, celui-ci est avantageusement présent à une concentration maximale de 20% en poids, et de préférence, comprise entre 0,1 et 10% en poids par rapport au poids total de la composition.

Comme 'polymère fixant' utilisable dans les compositions de mise en forme et/ou de coiffage et/ou de maintien des cheveux, on peut citer les polymères fixants anioniques, les polymères fixants cationiques, les polymères fixants non ioniques, les polymères fixants amphotères et leurs mélanges.

Comme exemple de polymères fixants anioniques, on peut citer ceux à groupements carboxyliques, tels que les homo- ou copolymères d'acide acrylique ou méthacrylique ou leurs sels, les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés, les copolymères d'acide crotonique, les copolymères d'acides ou d'anhydrides carboxyliques monoinsaturés en C₄-C₈, les polyacrylamides comportant des groupements carboxylates.

Comme exemples de polymères fixants cationiques, on peut citer :
(1) les copolymères d'acrylamide et de méthacrylate de diméthylaminoéthyle quaternisé au sulfate de diméthyle ou avec un halogénure de diméthyle, les copolymères d'acrylamide et de chlorure de méthacryloyloxyéthyltriméthylammonium ; le copolymère d'acrylamide et de méthosulfate de méthacryloyloxyéthyltriméthylammonium ; les copolymères vinylpyrrolidone/acrylate ou méthacrylate de dialkylaminoalkyle quaternisés ou non ;
(2) les polysaccharides cationiques, de préférence à ammonium quaternaire tel que les gommes de guar contenant des groupements cationiques trialkylammonium ;
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane .
(5) les dérivés de cellulose cationiques tels que les copolymères de cellulose ou de dérivés de cellulose greffés avec un monomère hydrosoluble comportant un ammonium quaternaire.

Les polymères fixants amphotères utilisables selon l'invention sont choisis parmi les polymères suivants :
(1) les copolymères à motifs vinyliques acides et à motifs vinyliques basiques ;
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Parmi les 'agents conditionneurs ou traitants' utilisables dans les compositions de coiffage ou de mise en forme des cheveux, on peut notamment utiliser des silicones volatiles ou non, linéaires ou cycliques et leurs mélanges, les polydiméthylsiloxanes, les polyorganosiloxanes quaternisés, les polyorganosiloxanes à groupements aminoalkyles modifiés par des groupements alcoxycarbonyalkyles, des polyorganosiloxanes tels que le copolymère polydiméthylsiloxane- polyoxyalkyle du type Diméthicone Copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Comme 'agent tensioactif' utilisable dans les compositions de l'invention, on peut citer un agent tensioactif de type non-ionique, anionique, cationique ou amphotère. En particulier pour les compositions capillaires, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Les compositions selon l'invention peuvent en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.
Par 'pigments', il faut comprendre des particules de toute forme, blanches ou colorées, minérales ou organiques, insolubles dans le milieu physiologique, destinées à colorer la composition.
Par 'nacres', il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.
Les paillettes peuvent être choisies parmi celles en résine acrylique, polyester, polyéthylène téréphtalate, en aluminium.

Comme 'agents épaississants' utilisables selon l'invention, on peut citer la cellulose et ses dérivés comme les éthers de cellulose, les hétérobiopolysaccharides tels que la gomme de xanthane, les scléroglucanes, les acides polyacryliques réticulés ou non. On peut citer par exemple :
- les polymères naturels tels que les gommes de xanthane et de guar ou encore les dérivés cellulosiques, les amidons et les alginates.
- les polymères synthétiques acryliques réticulés tels que les Carbopols® commercialisés par la société Goodrich et les polymères d'acide 2-acrylamido-2-méthylpropane-sulfonique (AMPS) réticulés et au moins partiellement neutralisés, comme par exemple le produit commercialisé sous la dénomination Hostacerin® AMPS par la société Clariant.
Les agents épaississants sont présents de préférence dans des proportions variant entre 0,1 et 5 % en poids environ par rapport au poids total de la composition.

Les compositions selon l'invention peuvent comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

Les compositions selon l'invention peuvent se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de pâte.
Pour une application sur les cheveux en particulier, la composition est généralement sous forme de solutions aqueuses, alcooliques ou hydroalcooliques; sous forme de crèmes, de lotions, de gels, d'émulsions, de mousses; sous forme de compositions pour aérosol comprenant également un agent propulseur sous pression.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

L'invention concerne donc en particulier une composition cosmétique de soin et/ou de maquillage des cils, caractérisée en ce qu'elle contient au moins une quantité efficace d'un extrait de bactérie tel que défini précédemment dans un milieu cosmétiquement acceptable comprenant au moins un polymère filmogène.
La composition peut comprendre en outre au moins un adjuvant cosmétique choisi parmi les huiles, les cires, les solvants organiques, les agents plastifiants, les agents tensioactifs, les charges, les pigments, les agents conférant de bonnes propriétés de recourbement des cils et leurs mélanges. Pour favoriser le recourbement des cils, on pourra utiliser une dispersion de particules multiphasées telle que décrite dans la demande WO03/007898, les particules multiphasées comprenant au moins une phase souple au moins en partie externe comportant au moins un polymère souple ayant au moins une température de transition vitreuse inférieure ou égal à 60°C et au moins une phase rigide au moins en partie interne, la phase rigide étant un matériau amorphe ayant au moins une température de transition vitreuse supérieure à 60°C, le polymère souple étant fixé au moins partiellement par greffage chimique sur la phase rigide.

Selon un mode particulier, la composition de soin et/ou de maquillage des cils est caractérisée en ce qu'elle est exemple de filtre UV. Par 'exempte de filtre UV', on entend une composition qui comprend moins de 0.1% de filtre UV, préférentiellement moins de 0.01% de filtre UV et encore plus préférentiellement aucun filtre UV.

De préférence, la composition sera un mascara. Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

L'invention porte également sur une composition cosmétique de soin et/ou de maquillage des ongles, caractérisée en ce qu'elle contient au moins une quantité efficace d'un extrait de bactérie tel que défini précédemment dans un milieu cosmétiquement acceptable comprenant de l'eau ou un mélange d'eau et de solvants organiques hydrophiles et au moins un polymère filmogène.
La composition pourra également comprendre un agent choisi parmi un agent plastifiant, un agent tensioactif, des charges, des pigments, des nacres et leurs mélanges.
En particulier, la composition pourra être : un produit de maquillage des ongles tel qu'un vernis à ongles coloré, une base pour les ongles ou "base-coat" en terminologie anglo-saxonne, une composition de finition, encore appelée "top-coat", à appliquer sous ou sur le produit de maquillage des ongles, un dissolvant pour vernis à ongles ou bien encore un produit de soin cosmétique des ongles tel qu'une base traitante destinée à protéger, à renforcer et/ou réparer les ongles.

L'invention couvre aussi une composition cosmétique de mise en forme et/ou de coiffage des cheveux, caractérisée en ce qu'elle contient au moins une quantité efficace d'un extrait de bactérie tel que défini précédemment dans un milieu cosmétiquement acceptable comprenant au moins agent choisi parmi un agent réducteur et un polymère fixant.
En particulier les compositions de déformation (mise en forme) des cheveux comprendront au moins un agent réducteur, et les compositions de coiffage des cheveux comprendront au moins un polymère fixant.
La composition pourra comprendre en outre au moins un agent choisi parmi un agent tensioactif, un polymère filmogène, un agent conditionneur ou traitant, des céramides, un agent épaississant, un agent propulseur pour le conditionnement en laques et leurs mélanges.
Selon un mode particulier, la composition de mise en forme et/ou de coiffage des cheveux est caractérisée en ce qu'elle est exempte de filtre UV. Par 'exempte de filtre UV', on entend une composition qui comprend moins de 0.1% de filtre UV, préférentiellement moins de 0.01% de filtre UV et encore plus préférentiellement aucun filtre UV.

La composition de mise en forme et/ou de coiffage des cheveux se présentera généralement sous la forme d'une composition capillaire pour déformation semi-permanente ou permanente, d'un shampooing ou après-shampooing pour cheveux permanentés, d'un gel ou d'une lotion de mise en plis, d'une lotion pour le brushing, d'une composition de fixation et de coiffage telles que les mousses, laques ou spray, ou bien sous la forme d'un mascara ou d'une laque de maquillage pour cheveux.

Préférentiellement, les compositions de l'invention comprennent un extrait cellulaire de bactérie filamenteuse non photosynthétique non fructifiante, tel que défini précédemment.

De préférence, les compositions selon l'invention sont caractérisées en ce que l'extrait de bactérie ou une fraction dudit extrait est en une quantité comprise entre 0.01% et 100% du poids total de la composition, préférentiellement entre 0.05 et 10% du poids total de la composition et encore plus préférentiellement entre 0.1 et 5% du poids total de la composition.

L'application desdites compositions sera réalisée par tout moyen adéquat, tel qu'un pinceau, une brosse, un spray ou les doigts par exemple.
Lorsque la composition sera sous la forme d'une solution de soin pour les ongles, l'application pourra se faire par simple trempage des doigts dans ladite solution.

Un autre objet de l'invention est un procédé cosmétique mise en forme et/ou de coiffage des cheveux, de soin et/ou de maquillage des cils ou des ongles, caractérisé en ce que l'on applique sur les cheveux et/ou les cils et/ou les ongles l'une des compositions telles que définies précédemment.

L'application des compositions selon l'invention se fera selon la technique d'utilisation habituelle desdites compositions. Par exemple :
- l'application sur cheveux peut avoir lieu avant (lotion, 1 heure avant), pendant (shampooing) ou après (lotions sprays) le shampooing ; la composition peut être appliquée sur cheveux secs (laques, sprays, lotions) ou sur cheveux humides (compositions de déformation permanente ou de mise en plis) ;
- l'application d'une base de soin sur les ongles pourra se faire avant ou après la pose du vernis ;
- la composition cosmétique de traitement des cils pourra être appliquée juste après application du mascara recourbant ; avantageusement, l'extrait de bactérie sera un composant du mascara recourbant lui-même.

Les compositions peuvent être appliquées quotidiennement ou à une fréquence de 2 à 3 applications par semaine, ceci pendant une durée de 1 à 3 mois, renouvelable selon le degré d'altération des matières kératiniques de l'individu à traiter. L'invention est illustrée plus en détail dans les exemples non limitatifs suivants.

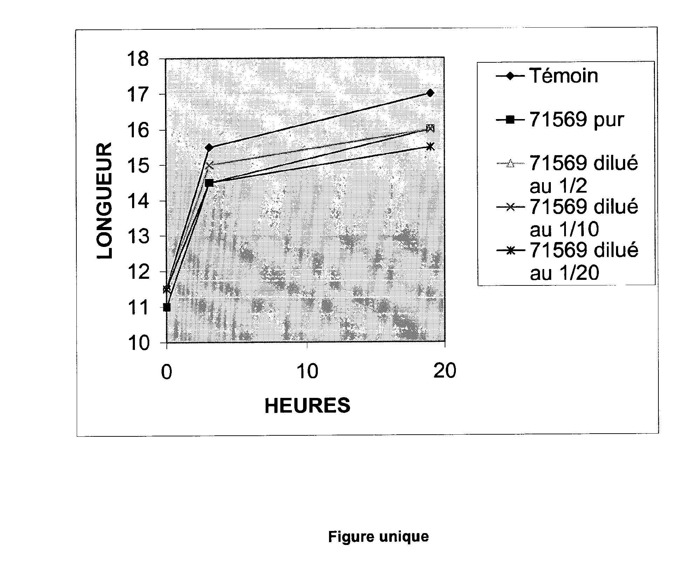

La figure représente la longueur des mèches suspendues après traitement avec l'extrait bactérien selon l'invention, en fonction du temps (heures).

### EXEMPLES

### Exemple 1 : Préparation des extraits de Vitreoscilla filiformis

La souche de *Vitreoscilla filiformis* (ATCC 15551) est mise en culture selon le procédé décrit dans la demande de brevet WO-A-93-00741. Il s'agit d'un procédé de culture en continu. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % V/V dans du milieu neuf durant 48 heures environ jusqu'à l'obtention d'une culture stable. Un Erlenmeyer de 1 litre contenant 200 ml de milieu neuf est alors ensemencé avec 4 ml de la culture précédente. La culture en Erlenmeyer s'effectue à 26°C sur une table de culture agitée à 100 tours/minutes. Le pied de cuve ainsi obtenu sert d'inoculum à un fermenteur de 50 litres. La croissance s'effectue à 26°C, pH 7, 100 tours/minute et pO₂ ≥ 15 %.
Après 30 heures de croissance, la biomasse est transférée dans un fermenteur de 3000 litres utiles, pour être cultivée dans les mêmes conditions. Après 48 heures de croissance on récolte les cellules en continu. La biomasse est alors concentrée 50 fois environ par centrifugation. Les cellules obtenues sont alors congelées au fur et à mesure de la culture continue. Ces cellules peuvent être utilisées en l'état après décongélation (extrait cellulaire non stabilisé) ou bien être stabilisées par autoclavage à 121°C durant 20 à 40 minutes (extrait cellulaire stabilisé). Les cellules ont alors éclaté durant la stérilisation en libérant le cytosol et en agglomérant les protéines ainsi que les parois. Le produit obtenu est alors biphasique.
La phase liquide surnageante peut être filtrée à 0,22 µm pour éliminer les particules ('surnageant').
L'extrait de bactérie, sous forme d'extrait cellulaire (stabilisé ou non) ou de surnageant, est utilisable en l'état (forme aqueuse) ou peut être lyophilisé suivant les techniques classiques (forme lyophilisée).

### Exemple 2 : Mise en évidence d'un effet renforçateur et embellisseur des ongles : amélioration de la dureté, de la vitesse de pousse, de l'épaisseur et de l'aspect général des ongles.

Un extrait de *Vitreoscilla filiformis* tel que préparé selon l'exemple 1 (extrait cellulaire, sous forme aqueuse) est appliqué sous une forme pure ou diluée (ex : 1/2, 1/10^{è} et 1/20^{è}) sur les ongles durant 1 à 5 minutes à température ambiante. L'application peut se faire par trempage des doigts dans l'extrait ou par application à l'aide d'un pinceau ou encore par application à l'aide d'un applicateur roll'on ou autres. Une main est traitée et l'autre main est non traitée : cette dernière constitue le témoin. Il est important de ne pas rincer le produit avant une heure après l'application.
Dès les premières applications, il est constaté un effet de renforcement des ongles en présence de l'extrait bactérien : ils sont qualifiés de plus durs et plus résistants à la cassure et/ou au dédoublement par rapport à la main témoin.
Après plusieurs applications, il est observé une amélioration de l'état des ongles.

Ces résultats ont été confirmés par une étude réalisée sur 30 sujets : 15 d'entre eux ont les ongles fragiles et mous, les 15 autres ont des ongles durs et cassants.
Le traitement est effectué sur une seule main, l'autre constituant le contrôle.
Les ongles de la main traitée sont trempés pendant 1 minute dans la solution aqueuse d'extrait cellulaire de *Vitreoscilla filiformis* sous forme pure telle que préparée à l'exemple 1, sans rinçage pendant au moins 30 minutes à 1 heure après application.
L'application est renouvelée quotidiennement pendant une période de 3 semaines, à l'issue de laquelle est réalisée une première évalution (J21), puis le traitement est reconduit pour 3 semaines avec une seconde évaluation à l'issue des 6 semaines (J42).
Ces évaluations consistent à :
- mesurer la vitesse de pousse de l'ongle traité par rapport à l'ongle témoin ;
- mesurer l'épaisseur de l'ongle traité par rapport à l'ongle témoin ;
- noter de 0 (effet -) à 9 (effet ++) l'efficacité du produit selon différents critères : dureté, brillance, aspect lisse, ongles dédoublés, ongles cassants, état général de l'ongle.

Pour les mesures de vitesse de pousse et d'épaisseur des ongles, l'analyse statistique est réalisée à l'aide du test t de Student.

Pour l'évaluation subjective de l'aspect général de l'ongle, le test utilisé est le test des rangs signés de Wilcoxon.

### a) vitesse de pousse

La vitesse de pousse des ongles est mesurée avant et après 3 et 6 semaines (J21 et J42) d'utilisation quotidienne du produit par analyse d'images réalisées au vidéomicroscope.

Le vidéomicroscope est un microscope mobile à fibre optique à objectif variable, couplé à un système informatique d'acquisition d'image (Puppin D et al., Journal of the American Academy of Dermatology, 1993, 28 : 923-927). L'objectif est mis directement devant la zone étudiée, sans contact et l'image est observée sur l'écran informatique.
Pour évaluer la vitesse de pousse de l'ongle, celui-ci est marqué d'une petite strie à sa surface à J0. L'avancée de cette strie sur l'ongle permet de calculer une vitesse de pousse.

Les tableaux ci-dessous présentent une synthèse des résultats individuels obtenus :

| Vitesse de pousse des ongles (mm/jour) | | |
|---|---|---|
| | Traité | Non traité |
| J21 | 0.133 ± 0.005 | 0.134 ± 0.004 |
| J42 | 0.132 ± 0.004 | 0.129 ± 0.004 |

| Variations | | |
|---|---|---|
| | J21 | J42 |
| % de variation | 0% | 4% |
| % des sujets présentant une augmentation | 43% | 66% |

La vitesse de pousse des ongles est donc augmentée après 21 jours sur 43% des sujets, et après 42 jours sur 66% des sujets, par rapport à un ongle non traité.

### b) épaisseur des ongles

L'épaisseur de l'ongle est mesurée par échographie ultrasonore à l'aide du DermaScan C® version 3 avant et après 6 semaines (J42) d'utilisation quotidienne du produit. Le principe est le suivant : un faisceau ultrasonore est émis par une céramique piézo-électrique et ce faisceau est partiellement réfléchi par les interfaces séparant deux milieux d'impédance ultrasonore différente (Wollina U. et al., Skin research and Technology, 2001, 7 : 60-64).
Cette méthode permet une évaluation bidimensionnelle de l'ongle et l'analyse des images permet de calculer l'épaisseur de l'ongle avec une précision estimée à 2%.

Les tableaux ci-dessous présentent une synthèse des résultats individuels obtenus :

| Epaisseur des ongles (mm) | | |
|---|---|---|
| | Traité | Non traité |
| J0 | 0.441 ± 0.009 | 0.440 ± 0.009 |
| J42 | 0.465 ± 0.010 | 0.441 ± 0.009 |

| Variations | |
|---|---|
| | J42 |
| % de variation | 5% |
| % des sujets présentant une amélioration | 79% |

Le produit augmente significativement l'épaisseur des ongles sur 79% des sujets à J42, par rapport à l'ongle non traité.

### c) aspect général de l'ongle

L'efficacité du produit à J0, J5, J21 et J42 est évaluée par les sujets sur la base de l'appréciation (échelle structurée de 0 à 9) des critères suivants : dureté, brillance, aspect lisse, ongles dédoublés, ongles cassants et état général de l'ongle.

Le tableau ci-dessous présente la synthèse des variations des résultats donnés par les volontaires :

| | J5 | J21 | J42 |
|---|---|---|---|
| Dureté de l'ongle | 0.5 ± 0.3 | 0.8 ± 0.3 | 1.4 ± 0.5 |
| Brillance des ongles | 0.7 ± 0.2 | 1.3 ± 0.3 | 1.8 ± 0.4 |
| Aspect lisse des ongles | 0.8 ± 0.2 | 0.8 ± 0.3 | 1.3 ± 0.4 |
| Ongles dédoublés | 1.4 ± 0.5 | 1.8 ± 0.7 | 2.9 ± 0.6 |
| Ongles cassants | 1.8 ± 0.5 | 2.2 ± 0.7 | 3.6 ± 0.5 |
| Etat général des ongles | 1.1 ± 0.3 | 2.2 ± 0.5 | 2.6 ± 0.5 |

Après 42 jours de traitement, les sujets ont constaté une nette amélioration de l'état de leurs ongles : ils sont plus durs, plus brillants, plus lisses, moins dédoublés, moins cassants et moins abîmés. L'amélioration est constatée dès les 5 premiers jours et se poursuit jusqu'à la fin du traitement.

L'ensemble de ces données montre que l'extrait cellulaire de *Vitreoscilla filiformis* permet de renforcer les ongles en augmentant leur dureté, leur vitesse de pousse, leur épaisseur et leur aspect global (plus lisse, plus brillant, moins dédoublé, moins cassant et moins abîmé).

### Exemple 3 : Mise en évidence d'un effet sur l'élasticité et la forme du cheveu

Des mèches standardisées de cheveux naturels (longueur 30 cm, masse 2.5g) sont immergées durant 10 minutes à T° ambiante (env. 21°C) dans 5 ml d'eau (témoin) ou d'extrait de *Vitreoscilla filiformis* (extrait cellulaire pur ou dilué : 1/2, 1/10^{è} et 1/20^{è}).
Les mèches sont ensuite rincées de manière standardisée : on effectue 15 passages des doigts le long des mèches sous flux continu d'eau du robinet à 30°C.
Les mèches sont ensuite enroulées sur bigoudis classiques (diamètre = 2cm; longueur 10 cm), puis mises à sécher sous casque de coiffage (t°=65°C) durant 30 minutes.
Une fois déroulées des bigoudis, elles sont ensuite suspendues verticalement et la longueur totale de chacune est enregistrée à T0 puis à différents intervalles de temps, par exemple 3 et 19 heures, afin de quantifier la vitesse de détente de ces mèches (retour à l'état de forme antérieur).
Une mèche traitée qui garde ainsi la longueur la plus courte pendant le temps le plus long possible rend ainsi compte de l'effet de tenue et de mise en forme du cheveu apporté par le produit étudié.
Les résultats de longeurs des mèches (cm) mesurées après traitement sont présentés dans le tableau ci-dessous et représentés graphiquement sur la figure annexée.

| HEURES | 0 | 3 | 19 |
|---|---|---|---|
| Témoin | 11,5 | 15,5 | 17 |
| 71569 pur | 11 | 14,5 | 16 |
| 71569 dilué au 1/2 | 11,5 | 15 | 16 |
| 71569 dilué au 1/10 | 11,5 | 15 | 16 |
| 71569 dilué au 1/20 | 11,5 | 14,5 | 15,5 |

Ces résultats montrent que l'extrait cellulaire de *Vitreoscilla filiformis* appliqué pur ou dilué sur une mèche de cheveu lui confère une meilleure tenue par rapport à un témoin (eau) : il permet de prolonger l'effet bouclé d'une mèche de cheveu, en augmentant son élasticité et en maintenant sa mise en forme. Cet effet n'est pas dose-dépendant, le résultat étant sensiblement équivalent en présence de l'extrait pur ou d'un extrait dilué au 1/20^{è}.

La même expérience a été reproduite avec le surnageant dudit extrait cellulaire : les résultats montrent également un effet sur la tenue de l'effet bouclé de la mèche par rapport au témoin (eau).

### Exemple 4 : Exemples de formulations

Les valeurs sont exprimées en % par rapport au poids total de la composition, sauf indication contraire.
Ces formulations sont préparées selon les méthodes usuelles.

### Base de soin renforçatrice des ongles

- nitrocellulose 15%
- plastifiant et résine 9%
- extrait cellulaire de *Vitreoscilla filiformis* (selon ex.1) 1%
- alcool éthylique 7%
- alcool isopropylique 7%
- eau 2%
- acétate de butyle 30%
- acétate d'éthyle qsp 100%

### Lotion non rincée pour cheveux permanentés

- extrait cellulaire de *Vitreoscilla filiformis* (selon ex. 1) 1%
- Acide linoléique 1%
- Propylène glycol 22,8%
- Ethanol 95° 55.1%
- Eau purifiée qsp 100%

### Mascara

- surnageant extrait de *Vitreoscilla filiformis* (forme lyophilisée) 1g
- cire de carnauba 20g
- Stéarate de glycéryle polyoxyéthyléné (30 OE) (Tagat S de la société GOLDSCHMIDT) 8g
- Oxyde de fer noir 5g
- Propylène glycol 5g
- Hydroxyéthylcellulose 2.5g
- Eau qsp 100g

Des compositions similaires pourront être préparées avec 5% d'extrait.

## Revendications

1. Utilisation dans une composition cosmétique, d'une quantité efficace d'au moins un extrait de bactérie filamenteuse non photosynthétique non fructifiante, en tant qu'agent améliorant la structure des matières kératiniques.

2. Utilisation selon la revendication 1, **caractérisée par le fait que** ledit extrait de bactérie est destiné à durcir les ongles et/ou augmenter leur résistance à la cassure et/ou au dédoublement.

3. Utilisation selon la revendication 1, **caractérisée par le fait que** ledit extrait de bactérie est destiné à augmenter l'épaisseur des ongles.

4. Utilisation selon la revendication 1, **caractérisée par le fait que** ledit extrait de bactérie est destiné à stimuler la pousse et/ou la repousse des ongles.

5. Utilisation selon la revendication 1, **caractérisée par le fait que** ledit extrait de bactérie est destiné à prolonger la tenue des vernis à ongles.

6. Utilisation selon la revendication 1, **caractérisée par le fait que** ledit extrait de bactérie est destiné à augmenter l'élasticité et/ou maintenir la mise en forme des cheveux, en particulier prolonger l'effet bouclé des cheveux.

7. Utilisation selon la revendication 1, **caractérisée par le fait que** ledit extrait de bactérie destiné à augmenter l'élasticité et/ou maintenir la mise en forme des cils, en particulier prolonger l'effet courbé du cil.

8. Utilisation selon l'une quelconque des revendications 1 à 7, **caractérisée en ce que** que ladite bactérie appartient au genre *Beggiatoa, Vitreoscilla, Flexithrix ou Leucothrix.*

9. Utilisation selon l'une quelconque des revendications 1 à 8, **caractérisée en ce que** ladite bactérie est une souche de *Vitreoscilla filiformis,* en particulier la souche ATCC15551.

10. Utilisation selon l'une quelconque des revendications 1 à 9, **caractérisée par le fait que** l'extrait de bactérie est un extrait cellulaire ou le surnageant dudit extrait, préférentiellement un extrait cellulaire ou une fraction active dudit extrait.

11. Utilisation selon l'une quelconque des revendications 1 à 10, **caractérisée en ce que** l'extrait de bactérie ou une fraction active dudit extrait est en une quantité comprise entre 0.01% et 100% du poids total de la composition, préférentiellement entre 0.05 et 10% du poids total de la composition et encore plus préférentiellement entre 0.1 et 5% du poids total de la composition.

12. Composition cosmétique de soin et/ou de maquillage des cils, **caractérisée en ce qu'**elle contient au moins une quantité efficace d'un extrait de bactérie tel que défini dans l'une quelconque des revendications 8 à 11 dans un milieu cosmétiquement acceptable comprenant au moins un polymère filmogène.

13. Composition cosmétique de soin et/ou de maquillage des ongles, **caractérisée en ce qu'**elle contient au moins une quantité efficace d'un extrait de bactérie tel que défini dans l'une quelconque des revendications 8 à 11 dans un milieu cosmétiquement acceptable comprenant de l'eau ou un mélange d'eau et de solvants organiques hydrophiles et au moins un polymère filmogène.

14. Composition cosmétique de mise en forme et/ou de coiffage et/ou de maintien des cheveux, **caractérisée en ce qu'**elle contient au moins une quantité efficace d'un extrait de bactérie tel que défini dans l'une quelconque des revendications 8 à 11 dans un milieu cosmétiquement acceptable comprenant en outre au moins un agent choisi parmi un agent réducteur et un polymère fixant.

15. Composition selon l'une quelconque des revendications 12 à 14, **caractérisée en ce que** ledit extrait de bactérie est un extrait cellulaire ou une fraction active dudit extrait.

16. Composition selon l'une quelconque des revendications 12 à 15, **caractérisée en ce que** l'extrait de bactérie ou une fraction active dudit extrait est en une quantité comprise entre 0.01% et 100% du poids total de la composition, préférentiellement entre 0.05 et 10% du poids total de la composition et encore plus préférentiellement entre 0.1 et 5% du poids total de la composition.

17. Procédé cosmétique de mise en forme et/ou de coiffage des cheveux, ou de soin et/ou de traitement des cils et/ou des ongles, **caractérisé en ce que** l'on applique sur les cheveux, les cils ou les ongles l'une des compositions telles que définies dans les revendications 12 à 16.
